# EUROPEAN PATENT APPLICATION

(11) **EP 4 297 037 A1**
(43) Date of publication of application: **27.12.2023**
(21) Application number: 22181091.4
(22) Date of filing: 24.06.2022
(51) Int. Cl.: G16B 40/00, G16B 20/00

(54) **DEVICE FOR DETERMINING AN INDICATOR OF PRESENCE OF HRD IN A GENOME OF A SUBJECT**

(71) Applicant: Seqone, 34000 Montpellier (FR)
(72) Inventor: PHILIPPE, Nicolas, 34150 Gignac (FR); BRUNEL, Samantha, 34000 Montpellier (FR); BEAUMEUNIER, Sacha, 34540 Balaruc le vieux (FR); DUFORET, Nicolas, 34670 Saint Bres (FR); RUZICKA, Jiri, 34000 Montpellier (FR); BERTAND, Denis, 34400 Lunel (FR); GOTTIN, Céline, 34080 Montpellier (FR)
(74) Representative: Icosa

(57) **Abstract**

The present invention relates to a device for determining an indicator of presence of Homologous Recombination Deficiency, HRD, in a genome of a subject, the device being configured for: receiving shallow WGS data and non-shallow sequencing data relative to a group of genes in the subject genome, obtaining at least one first parameter from the shallow WGS data and at least one second parameter from non-shallow sequencing data, and determining, by applying a Machine Learning Model to the obtained at least first and second parameters, an indicator of presence of HRD in the subject genome.

## Description

### FIELD OF INVENTION

The present invention relates to a method and a device for detecting Homologous Recombination Deficiency in the genome of a subject.

### BACKGROUND OF INVENTION

Homologous Recombination (HR) is a natural DNA repair mechanism. This mechanism may be broken by the inactivation of genes in the Homologous Recombination Repair (HRR) pathway. This inactivation is referred to as Homologous Recombination Deficiency (HRD) and leads to the accumulation of a large number of genomic alterations. This deficiency is associated with several tumor types, including breast, ovarian, prostate and pancreatic cancers.

It has been found that HRD tumors are more sensitive to some treatments, for example therapies based on poly(adenosine diphosphate [ADP]-ribose) polymerase (PARP) inhibitors (PARPi) for patients with ovarian cancers. Therefore, HRD identification is important for patients with some type of cancers, including ovarian cancers, since it makes it possible to choose appropriate treatments for patients.

There are tests in the prior art to determine HRD status (positive, i.e. with HRD, or negative, i.e. without HRD) of a subject. In particular, it is possible to determine whether a woman having an ovarian cancer has an HRD or not by estimating a triplet of measures using Single Nucleotide Polymorphism (SNP) sequencing data based on a custom hybridization capture panel. These measures are Large Scale Transitions (LST), Loss of Heterozygosity (LoH) and Telomeric Allelic Imbalance (TAI), all three being increased when the Homologous Recombination Repair mechanism is broken. From these measures, the signature of HRD in the genome can be quantified. A major drawback of this method is that it requires the use of specific capture panel probes to target the SNPs and to enrich these regions to high coverage (50X in average). This method is therefore time and cost consuming.

Another existing solution is based on deep learning methods originally designed for the field of computer vision to evaluate the probability of HRD. This solution uses Shallow WGS data as input for their deep learning method. It is recalled that Shallow WGS is a technology based on shotgun sequencing which is used to obtain whole genome sequences at very low coverage, and is usually more cost-effective than high coverage WGS. A major drawback of this method is that the deep learning models have to be trained over a very large set of data, which is not easy to obtain.

The aim of the invention is to propose a new method for identifying an HRD in a subject which overcomes the drawbacks of existing methods identified above.

### SUMMARY

This invention thus relates to a method and a system for automatically identifying HRD status of a subject by using a Machine Learning approach taking as input several kinds of genetic markers. These genetic markers may be obtained from both Shallow WGS data and panel data. By panel data, it is meant data derived from panel testing, i.e. from sequencing performed on a set of regions known to be associated with the development of a given disease (for example, regions comprising genes associated with ovarian cancer, such as BRCA1 or BRCA2). Typically, variants responsible for the disease are looked into this set of genes. According to the invention, the genetic markers contain metrics quantifying genomic instability linked to HRD, metrics on the genetic variants identified from panel data and/or level of amplification for a set of genes known to be HRD markers. All of these inputs are then used in a Machine Learning classifier trained to predict whether a sample is HRD positive or negative.

An object of the invention therefore relates to a device for determining an indicator of presence of Homologous Recombination Deficiency, HRD, in a genome of a subject. In one or several embodiments, the device may comprise:
- an input interface for receiving first sequencing data from a shallow Whole Genome Sequencing, WGS, process on the subject genome and second sequencing data from a non-shallow sequencing process on a group of segments in the subject genome, said group of segments comprising a set of genes called panel set of genes;
- at least one processor configured for:
   o obtaining, from the first sequencing data, at least one first parameter among:
      ▪ a first measurement representative of a number of Large-scale State Transitions, LSTs, in the subject genome;
      ▪ a second measurement representative of a number of segments in the subject genome presenting a loss of a chromosome portion; and
      ▪ at least one indicator, called CNV indicator, representative of levels of amplification of genes among a predefined set of genes called CNV set of genes;
   o obtained, from the second sequencing data, at least one indicator, called panel indicator, representative of somatic variants on genes of the panel set of genes;
   o determining, from a trained Machine Learning model applied to the at least first parameter and the at least one panel indicator, an indicator of presence of HRD in the subject genome.

By "sequencing data from a non-shallow sequencing process", it is meant sequencing data obtained by a process which is not a shallow process, but a standard sequencing process, having typically an average coverage between 50X and 500X, for example a Next-Generation Sequencing (NGS) process. In addition, the "second sequencing data from a non-shallow sequencing process on a group of segments in the subject genome", which means that these non-shallow sequencing data are obtained by sequencing only a group of segments of the subject genome, and not the whole genome. The non-shallow sequencing process is therefore not a WGS process.

The "first measurement representative of a number of Large-scale State Transitions, LSTs, in the subject genome" may also be referred to as Large-scale Genomic Alteration (LGA). The "second measurement representative of a number of segments in the subject genome presenting a loss of a chromosome portion" may also be referred to as indicator of a level of Loss of Heterozygosity (LOH). These measurements are known by the person skilled in the art.

The above device makes it possible to determine an indicator of presence of an HRD in the subject genome from shallow WGS data and from non-shallow sequencing data on only a group of segments of the genome, and not the whole genome. Therefore, the HRD determination according to the present invention is more effective in terms of time and cost than in the prior art.

The measurements and/or indicators may be received by the device (e.g. from a sequencing device), or determined by the processor of the device from the received sequencing data.

In one or several embodiments, the at least one processor may be configured for obtaining the first measurement, the second measurement and the at least one CNV indicator, and wherein the Machine Learning model is applied to first measurement, the second measurement, the at least one CNV indicator and the at least one panel indicator.

According to these embodiments, all the measures and indicators are used as inputs of the Machine Learning model.

In one or several embodiments, the panel set of genes and the CNV set of genes may be mutually exclusive.

By "mutually exclusive", it is meant that the two sets have no gene in common. The panel set and the CNV set advantageously comprise genes implied in the HRD process. Defining or constricting these sets such as they are mutually exclusive reduces redundancy in data processing, making the HRD determination more cost and time efficient.

In one or several embodiments, the CNV set of genes may comprise genes among: AKT1, BARD1, CCNE1, EMSY, ESR1, H2AX, MRE11, PTEN, RAD51B, RAD52, RAD54.

In one or several embodiments, the panel set of genes may comprise BRCA1 and/or BRCA2 genes.

All of these genes are known to have a role in the HRD mechanism.

In one or several embodiments, the first measurement representative of the number of LSTs in the subject genome may be determined based on a number of pairs of adjacent segments, each segment of each pair being at least 10 Mb long, the segments of each pair having different Copy Numbers, CNs.

In one or several embodiments, the second measurement representative of the number of segments in the subject genome presenting a loss of a chromosome portion may be determined based on a number of genomic segments of at least 10 Mb long and having a Copy Number between 0.5 and 1.5.

In one or several embodiments, the at least one CNV indicator may comprise at least one of:
- for each gene of the CNV set of genes, a respective Copy Number of the gene;
- for each gene of the CNV set of genes, a binary value indicating whether a Copy Number Variation, CNV, is detected for the gene;
- each gene of the CNV set of genes, a ratio between the Copy Number of the gene and an average Copy Number of the gene in all the CNV set of genes;
- a number of genes among the CNV set of genes for which a CNV is detected.

In one or several embodiments, the at least one panel indicator may comprise a counter representing a number of genes having somatic variants among the panel set of genes.

In one or several embodiments, the subject suffers from a pathology, and the at least one processor may be further configured for:
- determining, based on at least one classification of somatic variants, genes having respective pathogenicity levels for said pathology above a predefined pathogenicity threshold;
and the panel set of genes may comprise said determined genes.

In one or several embodiments, the genes of the panel set of genes may be classified into several categories of pathogenicity level, wherein the at least one panel indicator comprises, for each category of pathogenicity level, a respective counter representing a number of genes of said each category of pathogenicity level having somatic variants.

In one or several embodiments, the indicator of presence of HRD in the subject genome may be a binary variable indicating whether an HRD is present or not in the subject genome.

In one or several embodiments, the at least one processor may be further configured for determining, from the trained Machine Learning model applied to the at least first parameter and the at least one panel indicator, a probability of presence of an HRD in the subject genome.

In one or several embodiments, the trained Machine Learning model may be a regression model.

For example, the trained Machine Learning model may be a regression model based on an elastic net regularization. Of course, other Machine Learning models may be used.

In one or several embodiments, the device may comprise another processor configured for training the Machine Learning model from a training dataset obtained from first sequencing data from shallow Whole Genome Sequencing, WGS, process on genomes of a plurality of subjects and second sequencing data from a non-shallow sequencing process on a group of segments of the genomes of the plurality of subjects, said group of segments comprising a set of genes called panel set of genes. The training dataset may comprise a plurality of training data subsets, each subset being respectively associated with a subject of the plurality of subjects and comprising:
- at least one respective first parameter determined from the first sequencing data among:
   o a respective first measurement representative of a number of Large-scale State Transitions, LSTs, in the genome of said subject;
   o a second measurement representative of a number of segments in the genome of said subject presenting a loss of a chromosome portion; and
   o at least one indicator, called CNV indicator, representative of levels of amplification of genes among a predefined set of genes called CNV set of genes;
- at least one respective indicator, called panel indicator and determined from the first the second sequencing data, representative of somatic variants on genes of the panel set of genes.

In one or several embodiments, the Machine Learning model may be trained on a fully supervised manner.

### DEFINITIONS

In the present invention, the following terms have the following meanings.

By "Whole genome sequencing (WGS)", or "full genome sequencing", "complete genome sequencing" or "entire genome sequencing", it is meant a process of determining the entirety, or nearly the entirety, of the DNA sequence of an organism's genome at a single time.

By "shallow WGS", it is meant a WGS process with very low coverage, for example an average coverage between 0.1X and 1X (preferably between 0.3X and 1X).

By "panel data", it is meant sequencing data obtained by traditional sequencing methods (e.g. high coverage sequencing) on specific regions of the genome. For example, these specific regions of the genome are regions comprising genes known to be associated with a given pathology. The term "panel data" is therefore opposed to "WGS data", which designates sequencing data representing the entire genome.

### BRIEF DESCRIPTION OF THE DRAWINGS

**Figure 1** is an example of a flow-chart representing a method for detecting the presence of an HRD in a subject according to embodiments of the invention;
**Figure 2** illustrates an example of a device for detecting the presence of an HRD in a subject according to embodiments of the invention.

### DETAILED DESCRIPTION

Expressions such as "comprise", "include", "incorporate", "contain", "is" and "have" are to be construed in a non-exclusive manner when interpreting the description and its associated claims, namely construed to allow for other items or components which are not explicitly defined also to be present

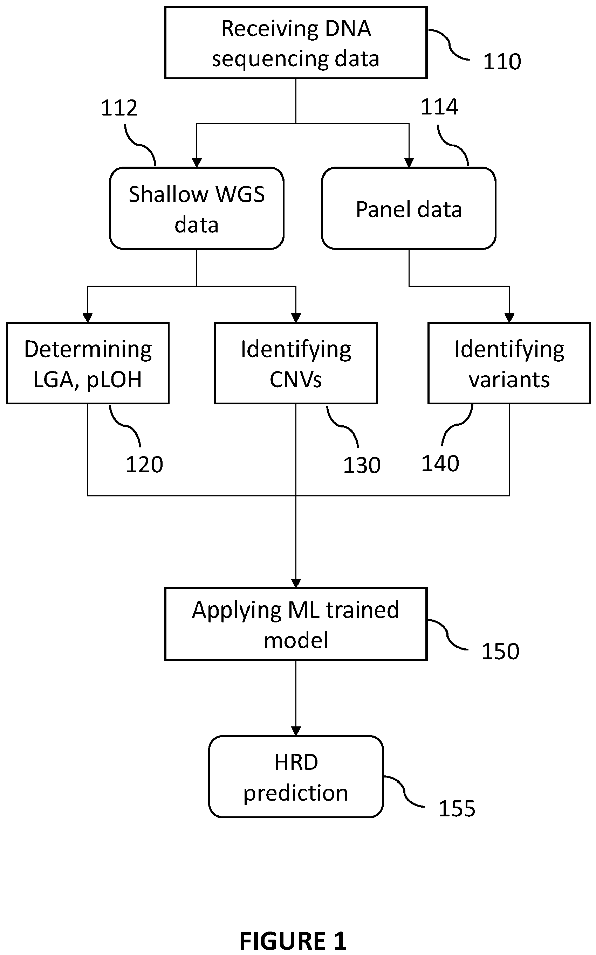

**Figure 1** is an example of a flow-chart representing a method for detecting the presence of an HRD in a subject according to embodiments of the invention.

At step 110, DNA sequencing data relative to a subject having a pathology associated with a tumor (for example, sequencing data of a women having an ovarian cancer) is received. This sequencing data may typically comprise shallow WGS data 112 and panel data 114. Shallow WGS data 112 corresponds to sequencing data obtained by a low coverage WGS process (e.g. process having an average coverage of at most 1X or 1.5X). Panel data 114 corresponds to data obtained by sequencing specific portions of the genome (i.e. specific DNA portions) by a classical sequencing process (having typically an average coverage between 50X and 500X), for example a Next-Generation Sequencing (NGS) process, said specific portions comprising genes pre-selected as being of interest for the type of cancer tumor.

According to one or several embodiments, two metrics may be determined (step 120) from shallow WGS data 112: a first metric quantifying the rate of chromosome breakages in the genome, and a second metric quantifying the rate of loss of heterozygosity in the genome.

In one or several embodiments, the first metric quantifying the rate of chromosome breakages in the genome may be an LGA (Large-scale Genomic Alteration) measurement. LGA represents the number of Large-scale State Transitions (LSTs) of the genome. It is recalled that LST is defined as a chromosomal break between adjacent regions of at least 10 megabases (Mb). In embodiments, LGA is determined by counting the number of genomic breaks, each genomic break corresponding to two adjacent genomic segments of at least 10 Mb long having different Copy Numbers (CNs) or, equivalently, different CN ratios (a CN ratio being defined as the CN of the genomic segment of the tested sample divided by the tumor ploidy). To this end, a value indicating the minimal CN ratio difference between two consecutive genomic segments may be computed from the shallow WGS data, and it may be considered that a genomic break is present is this value is in a predefined range, e.g. between 0.05 and 0.4. In embodiments, the number of genomic breaks may be determined after filtering out genomic segments shorter than 3 Mb.

It is noted that the CN of the genomic regions determined from the sequencing data are actually estimated values of the CN of the genomic region. In this specification, the term "CN" may be used for designating estimated CN.

In one or several embodiments, the second metric quantifying the rate of loss of heterozygosity in the genome may be a pLOH (pseudo-Loss of Heterozygosity) measurement. Loss of heterozygosity (LOH) corresponds to the loss of one parent's contribution to the cell, which may be caused by direct deletion, deletion due to unbalanced rearrangements, gene conversion, mitotic recombination, or loss of a chromosome portion. The number of genomic regions presenting an LOH cannot be determined based on shallow WGS data. Therefore, the invention proposes to approximate this number by a pLOH measurement, defined as the number of genomic segments on which a loss of a chromosome portion is detected. For example, the pLOH measurement may be the number of genomic segments of at least 10 Mb and having an estimated CN around 1 (e.g., between 0.5 and 1.5), or a CN ratio of around 0.5 (e.g., between 0.25 and 0.75).

Therefore, in one or several embodiments, the determination 120 of the two metrics may comprise a preliminary step of determining the global CN ratio profile of the genome from the shallow WGS data. In embodiments, this determination may be performed only when the average coverage of shallow WGS data is greater than a predefined threshold, for example 0.3X, to ensure sufficient quality of the data to be analyzed.

In addition, shallow WGS data may be used to determine (step 130) the presence of Copy Number Variations (CNVs) in a predefined first set of genes, by estimating the CNs of these genes. It is recalled that a CNV refers to the fact that the CNs of a gene or a portion of a gene in the genome differs from a subject to another. This variation may typically be due to deletions or amplifications of DNA segments. Many of the CNVs have no effect on health, but some may be associated with diseases.

In the present invention, the first set of genes for which a CNV determination (step 130) is carried out corresponds to genes associated with the pathology studied. For example, in the context of tumors associated with the presence of HRD (e.g. in ovarian, breast, prostatic or pancreatic cancers), the first set of genes may comprise up to eleven genes, chosen among: AKT1, BARD1, CCNE1, EMSY, ESR1, H2AX, MRE11, PTEN, RAD51B, RAD52, RAD54. All of these genes are indeed known for their role in HRD process. Of course, depending on the evolution of knowledge in genetics, this first set is likely to evolve. Also, some genes may be more or less relevant depending on the sex of the individual and/or the location of the tumor (breast vs ovaries for example). In some embodiments of the invention, the CNV determination (step 130) is performed for all of these eleven genes. In alternative embodiments, CNV determination (step 130) is performed only for a subset of this first set of genes.

In one or several embodiments, step 130 may comprise, for genes of the predefined first set of genes (all the predefined first set or only a subset of the predefined first set), a determination of an indicator relative to a level of amplification of a respective gene. For example, for each gene, this indicator may be a CN of the gene, or a binary value indicating whether a CNV is present for the gene, or a ratio between the CNs of the gene in the sample and an average CNs the average CN of the sample genome. Alternatively, this indicator may be a number of genes among the first set or the subset of the first set of tested genes for which a CNV is detected.

In one or several embodiments, variants present on a second set of genes may be determined (step 140) from panel data 114. This second set of genes (also called "panel set of genes") corresponds to genes belonging to portions for which panel data 114 have been obtained, i.e. for the portions of the genome for which classical sequencing process have been performed. This second set of genes comprises genes of interest for the type of cancer tumor studied. Advantageously, genes of the first and the second sets are mutually exclusive, which means that the second set of genes comprises genes that are not in the first set of genes.

For example, in the context of tumors associated with the presence of HRD (e.g. in ovarian, breast, prostatic or pancreatic cancers), the second set of genes may comprise BRCA1 and BRCA2, known for their role in HRR process. The second set of genes may also comprise genes known to have a certain pathogenicity (e.g. a pathogenicity probability above a predetermined threshold) according to biological and clinical reference or proprietary classifications of somatic variants, which may also provide clinical interpretations and annotations for somatic variants. These annotations may comprise for example a respective pathogenicity probability of the variant for a given pathology.

In one or several embodiments, the somatic variants identified in step 140 may be Indels, Single Nucleotide Variants (SNVs) and/or Multi-Nucleotide Variants (MNVs). Also, the somatic variants identified in step 140 may be short variants, i.e. variants concerning a small length of DNA (e.g. less than 10 base pairs).

In one or several embodiments, identification of somatic variants (step 140) may comprise one or more indicators of presence of variants having a level of pathogenicity among a predefined threshold. In particular, it may comprise the determination of a number of variants associated with a predefined class of pathogenicity. For example, 3 classes of pathogenicity may be defined: pathogenic, likely pathogenic and variants of uncertain significance (VUS). Such repartition of pathogenicity into classes is known by the person skilled in the art and the belonging of a given variant to one of the classes can be determined from reference or proprietary classifications. From these classes, 3 respective indicators may be defined, each representing a number of variants in the second set of genes belonging to a respective class. In embodiments, genes BRCA1 and BRCA2 may be considered apart from the other genes of the second set of genes, and a fourth indicator may be defined as the number of variants in these BRCA1 and BRCA2 genes. Alternatively, a fourth indicator and a fifth indicator may be defined, corresponding respectively to the numbers of variants in BRCA1 gene and in BRCA2 gene. In some embodiments, these numbers may represent the number of variants of the BRCA1 and/or 2 genes classified as pathogenic, or as classified as (pathogenic or likely pathogenic), etc.

It is noted that steps 120, 130 and 140 may be performed in parallel or consecutively, in any order.

Once the metrics / indicators have been obtained at steps 120, 130 and 140, these metrics / indicators are provided (step 150) as input data to a trained Machine Learning (ML) model, to obtain as output a predicted status 155 of the subject regarding HRD (for example, HRD positive or HRD negative). Alternatively or in complement, the output of the ML model may be a probability of presence of an HRD for the subject. In embodiments, if the probability of presence of an HRD is between 0.5 plus or minus a predefined threshold (e.g. 0.2), the result may be considered as "failed", since the probability is too close to 0.5 to confirm or rule out the presence of an HRD.

It is noted that the method for determining the presence of an HRD in a subject of **Figure 1** may be performed from only part of the metrics / indicators described above. For example, in some embodiments, the LGA and/or the pLOH may not be used as inputs of the ML model. In these embodiments, step 120 is therefore carried out only in part or is completely skipped. In other embodiments, only one or two of the metrics determined in step 120 from shallow WGS data 112 and the indicator(s) determined at step 140 from the panel data 114 are used. In such embodiments, step 130 is skipped.

In one or several embodiments, the ML model may have previously been trained in a fully-supervised manner, from a set of sequencing data corresponding to tumor samples for which the real status of the subject (HRD positive or negative) is known. For example, the real status may correspond to the result of a reference HRD test of the prior art considered to be the gold standard.

In one or several embodiments, the ML model may relate on regression algorithms, for example ridge regressions (based on the L2 norm), lasso regression (based on the L1 norm), or elastic net regularization (which combines L1 and L2 penalties of the lasso and ridge algorithms). Other ML algorithms may be used, including regression or classification algorithms, such as ensemble models, support vector machines, neural networks, etc. Such ML models are known and are not further detailed in this specification.

As mentioned above, the ML model may be trained in a preliminary stage from a set of sequencing data corresponding to tumor samples for which the real status is known. The sequencing data may typically comprise shallow WGS data and panel data. In some embodiments, a filtering on the data quality may be applied to keep only samples with a sufficient coverage (e.g. at least 0.1X for shallow WGS data) and/or a sufficient percentage of properly mapped reads (e.g. at least 50%, for both shallow WGS data and panel data). Also, when the status of a sample cannot be known by the gold standard, for example due to low tumoral cells or DNA yield, this sample may be discarded and therefore not used for training the ML model.

Metrics and indicators may then be determined from the sequencing data as detailed above (i.e. in a similar way than during the running phase), to be used as training data for the ML model. Due to the nature of the model, and the differences in the inputs, a normalization on the dataset may be performed to account for differences in variances. Before the learning of the ML model, each input group may be set to a zero mean and a unit variance. The ML model may then be trained using an optimization algorithm, for example a SAGA algorithm.

To evaluate the capacity of the above method to detect subjects with HRD, a leave-one-out cross validation approach has been used. The ML model was trained using training data from 63 women having an ovarian cancer. The training data comprised all of the metrics / indicators detailed above. Among the 63 women, 56 women obtained similar results than the Gold Standard, which represents an accuracy of the new method of Figure 1 around 89%. It is noted that the Gold Standard itself may eventually produce false positive or false negative results. Therefore, among the 7 patients having obtained divergent HRD results between the new method and the Gold Standard, some of them may actually correspond to Gold Standard errors, which would further increase the accuracy of the new method of HRD determination.

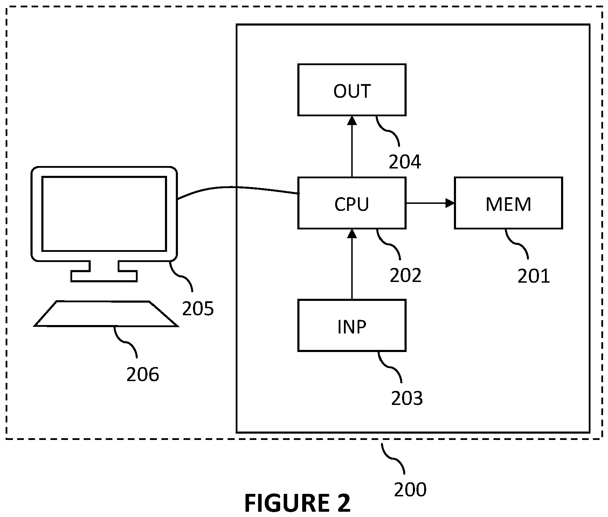

**Figure 2** illustrates an example of a device for detecting the presence of an HRD in a subject according to embodiments of the invention.

In these embodiments, the device 200 comprises a computer, this computer comprising a memory 201 to store program instructions loadable into a circuit and adapted to cause a circuit 202 to carry out steps of the method of **Figure 1** when the program instructions are run by the circuit 202. The memory 201 may also store data and useful information for carrying the steps of the present invention as described above, in particular the trained ML model used for predicting an HRD status of a subject.

The circuit 202 may be for instance:
- a processor or a processing unit adapted to interpret instructions in a computer language, the processor or the processing unit may comprise, may be associated with or be attached to a memory comprising the instructions, or
- the association of a processor / processing unit and a memory, the processor or the processing unit adapted to interpret instructions in a computer language, the memory comprising said instructions, or
- an electronic card wherein the steps of the invention are described within silicon, or
- a programmable electronic chip such as a FPGA chip (for « Field-Programmable Gate Array »).

The computer may also comprise an input interface 203 for the reception of sequencing data of the subject and an output interface 204 to provide the HRD status of the subject.

To ease the interaction with the computer, a screen 205 and a keyboard 206 may be provided and connected to the computer circuit 202.

Furthermore, the flow chart represented in **Figure 1** can represent all or part of the steps of a program which may be executed by a processor located in the transmitter. As such, **Figure 1** may correspond to the flow chart of the general algorithm of a computer program within the meaning of the invention.

A person skilled in the art will readily appreciate that various parameters disclosed in the description may be modified and that various embodiments disclosed may be combined without departing from the scope of the invention. Of course, the present invention is not limited to the embodiments described above as examples. It extends to other variants.

## Claims

1. A device for determining an indicator of presence of Homologous Recombination Deficiency, HRD, in a genome of a subject, the device comprising:
- an input interface for receiving first sequencing data from a shallow Whole Genome Sequencing, WGS, process on the subject genome and second sequencing data from a non-shallow sequencing process on a group of segments in the subject genome, said group of segments comprising a set of genes called panel set of genes;
- at least one processor configured for:
o obtaining, from the first sequencing data, at least one first parameter among:
▪ a first measurement representative of a number of Large-scale State Transitions, LSTs, in the subject genome;
▪ a second measurement representative of a number of segments in the subject genome presenting a loss of a chromosome portion; and
▪ at least one indicator, called CNV indicator, representative of levels of amplification of genes among a predefined set of genes called CNV set of genes;
o obtaining, from the second sequencing data, at least one indicator, called panel indicator, representative of somatic variants on genes of the panel set of genes; and
o determining, from a trained Machine Learning model applied to the at least first parameter and the at least one panel indicator, an indicator of presence of HRD in the subject genome.

2. The device of claim 1, wherein the at least one processor is configured for obtaining the first measurement, the second measurement and the at least one CNV indicator, and wherein the Machine Learning model is applied to first measurement, the second measurement, the at least one CNV indicator and the at least one panel indicator.

3. The device of any one of claims 1 and 2, wherein the panel set of genes and the CNV set of genes are mutually exclusive.

4. The device of any one of the preceding claims, wherein the CNV set of genes comprises genes among: AKT1, BARD1, CCNE1, EMSY, ESR1, H2AX, MRE11, PTEN, RAD51B, RAD52, RAD54.

5. The device of any one of the preceding claims, wherein the panel set of genes comprises BRCA1 and/or BRCA2 genes.

6. The device of any one of the preceding claims, wherein the first measurement representative of the number of LSTs in the subject genome is determined based on a number of pairs of adjacent segments, each segment of each pair being at least 10 Mb long, the segments of each pair having different Copy Numbers, CNs.

7. The device of any one of the preceding claims, wherein the second measurement representative of the number of segments in the subject genome presenting a loss of a chromosome portion is determined based on a number of genomic segments of at least 10 Mb long and having a Copy Number between 0.5 and 1.5.

8. The device of any one of the preceding claims, wherein the at least one CNV indicator comprises at least one of:
- for each gene of the CNV set of genes, a respective Copy Number of the gene;
- for each gene of the CNV set of genes, a binary value indicating whether a Copy Number Variation, CNV, is detected for the gene;
- for each gene of the CNV set of genes, a ratio between the Copy Number of the gene and an average Copy Number of the gene in all the CNV set of genes;
- a number of genes among the CNV set of genes for which a CNV is detected.

9. The device of any one of the preceding claims, wherein the at least one panel indicator comprises a counter representing a number of genes having somatic variants among the panel set of genes.

10. The device of any one of the preceding claims, wherein the subject suffers from a pathology, wherein the at least one processor is further configured for:
- determining, based on at least one classification of somatic variants, genes having respective pathogenicity levels for said pathology above a predefined pathogenicity threshold;
wherein the panel set of genes comprises said determined genes.

11. The device of any one of the preceding claims, wherein the genes of the panel set of genes are classified into several categories of pathogenicity level, wherein the at least one panel indicator comprises, for each category of pathogenicity level, a respective counter representing a number of genes of said each category of pathogenicity level having somatic variants.

12. The device of any one of the preceding claims, wherein the indicator of presence of HRD in the subject genome is a binary variable indicating whether an HRD is present or not in the subject genome.

13. The device of any one of the preceding claims, wherein the at least one processor is further configured for determining, from the trained Machine Learning model applied to the at least first parameter and the at least one panel indicator, a probability of presence of an HRD in the subject genome.

14. The device of any one of the preceding claims, wherein the trained Machine Learning model is a regression model.

15. The device of any one of the preceding claims, comprising another processor configured for training the Machine Learning model from a training dataset obtained from first sequencing data from shallow Whole Genome Sequencing, WGS, process on genomes of a plurality of subjects and second sequencing data from a non-shallow sequencing process on a group of segments of the genomes of the plurality of subjects, said group of segments comprising a set of genes called panel set of genes; wherein the training dataset comprises a plurality of training data subsets, each subset being respectively associated with a subject of the plurality of subjects and comprising:
- at least one respective first parameter obtained from the first sequencing data among:
∘ a respective first measurement representative of a number of Large-scale State Transitions, LSTs, in the genome of said subject;
∘ a second measurement representative of a number of segments in the genome of said subject presenting a loss of a chromosome portion; and
∘ at least one indicator, called CNV indicator, representative of levels of amplification of genes among a predefined set of genes called CNV set of genes;
- at least one respective indicator, called panel indicator and obtained from the first the second sequencing data, representative of somatic variants on genes of the panel set of genes.

16. The device of any one of the preceding claims, wherein the Machine Learning model is trained on a fully supervised manner.
